# EUROPEAN PATENT SPECIFICATION

(11) **EP 1 447 055 B1**
(45) Date of publication and mention of the grant of the patent: **07.06.2006**
(21) Application number: 04250515.6
(22) Date of filing: 30.01.2004
(51) Int. Cl.: A61B 19/00, A61B 17/17

(54) **Guidance system for rotary surgical instrument**
Führungssystem für rotatorisches chirurgisches Instrument
Système de guidage pour instrument chirurgical rotatif

(30) Priority: 04.02.2003 US 357592
(43) Date of publication of application: 18.08.2004
(73) Proprietor: Zimmer Technology, Inc., Chicago, Illinois 60606 (US)
(72) Inventor: McGinley, Shawn E., Fort Wayne IN 46814 (US); Grimm, James E., Winona Lake IN 46590 (US)
(74) Representative: Mays, Julie

(56) References cited:
- US-A1- 2002 107 518
- US-B1- 6 203 543
- US-B1- 6 450 978

## Description

### BACKGROUND OF THE INVENTION

### 1. Field of the Invention.

The present invention relates to computer assisted surgical navigation systems and, more specifically, to the use of a rotary instrument in a computer assisted surgical navigation system.

### 2. Description of the Related Art.

The controlled positioning of surgical instruments is of significant importance in many surgical procedures and various methods and guide instruments have been developed for properly positioning a surgical instrument. Such methods include the use of surgical guides which function as mechanical guides for aligning reamers, awls and other drilling and rotating instruments. The use of such surgical guides is common in orthopedic surgical procedures and such guides may be used to properly align a drill or other instrument with respect to a bone when preparing the bone for receiving an implant such as an artificial joint.

Computer assisted surgical navigation systems which provide for the image guidance of a surgical instrument are also known. Examples of various computer assisted navigation systems which are known in the art are described in U.S. Pat. Nos. 5,682,886; 5,921,992; 6,096,050; 6,348,058 B1; 6,434,507 B1; 6,450,978 B1; 6,490,467 B1; and 6,491,699 B1. Image guidance techniques typically involve acquiring preoperative images of the relevant anatomical structures and generating a data base which represents a three dimensional model of the anatomical structures. The relevant surgical instruments typically have a known and fixed geometry which is also defined preoperatively. During the surgical procedure, the position of the instrument being used is registered with the anatomical coordinate system and a graphical display showing the relative positions of the tool and anatomical structure may be computed in real time and displayed for the surgeon to assist the surgeon in properly positioning and manipulating the surgical instrument with respect to the relevant anatomical structure. It is also known in such computer assisted navigation systems to provide a guide for a rotary shaft that includes an array mounted on the guide for registering the guide in the coordinate system of the navigation system.

### SUMMARY OF THE INVENTION

The present invention provides a rotary surgical instrument which can be used with a computer assisted navigation system. A mounting assembly is provided that has at least one reference element registerable in the computer assisted navigation system. The mounting assembly is rotatable relative to the instrument. For example, a rotating shaft may extend through a cylindrical opening in the mounting assembly. The mounting assembly is biased so that the reference element is positioned in a desired orientation during operation of the instrument. For example, the mounting assembly may include a counterweight positioned opposite the reference element whereby the reference element is gravitationally biased toward a position above the rotational axis of the mounting assembly. This can be particularly useful in a computer assisted navigational system that the reference elements be within the line of sight of the sensors tracking the movement of the reference elements such as an optical tracking system.

The invention comprises, in one form thereof, a surgical instrument for use in a computer assisted navigation system. The instrument includes at least one reference element registerable in the computer assisted navigation and a mounting assembly defining an axis and rotatably mounted on the instrument. The reference element is positionable on the mounting assembly in a predetermined location which defines a first angular position relative to the axis. A counterweight is disposed on the mounting assembly and is radially outwardly spaced from the axis. The counterweight defines a second angular position relative to the axis and the first and second angular positions are separated by at least 90 degrees.

The at least one reference element may take the form of at least three non-linearly positioned reference elements. The mounting assembly may also include a radially outwardly extending mounting stem that is disposed substantially diametrically opposite the counterweight relative to the axis with the at least one reference element being mountable on a radially distal end of the mounting stem. The mounting assembly may also include a sleeve portion defining a cylindrical opening with the counterweight being integrally formed with the sleeve portion. A rotary member having a cylindrical shaft portion may be rotationally engaged with the mounting assembly.

The invention comprises, in another form thereof, a surgical instrument for use in a computer assisted navigation system. The instrument includes a rotary member having first and second opposed ends and a mounting assembly operably coupled to the rotary member wherein the rotary member and the mounting assembly are relatively rotatable about an axis. At least one reference element registerable in the computer assisted navigation system is disposed on the mounting assembly at a predetermined location. The mounting assembly defines a center of gravity that is spaced radially outwardly from the axis. The predetermined location of the reference element defines a first angular position relative to the axis and the center of gravity defines a second angular position relative to the axis wherein the first and second angular positions are separated by at least 90 degrees.

The mounting assembly may include a sleeve portion defining a cylindrical opening with the rotary member rotationally disposed within the cylindrical opening. The mounting assembly has a counterweight portion disposed radially outwardly from the axis wherein the at least one reference element is disposed substantially diametrically opposite the counterweight portion relative to the axis.

A rotational driver may be detachably secured to the first end of the shaft and a rotatable tool detachably secured to the second end. A collet assembly may also be disposed at the second end. The collet assembly may include a collet and a biasing member wherein the collet defines a central void and has a plurality of fingers biasable inwardly relative to the void. The biasing member is biasingly engageable with the plurality of collet fingers and is securable in a position biasing the plurality of fingers inwardly relative to the central void. A surgical tool having a shank may be inserted into the central void wherein the shank is rotationally fixedly engageable by the inwardly biasable plurality of collet fingers. The second end of the shaft may be defined by a cylindrical shaft having exterior threads and an axially disposed opening. The collet is partially positioned in the opening and at least a portion of the plurality of collet fingers projects from the opening. The biasing member threadingly engages the exterior threads and circumscribes the projecting portion of the plurality of collet fingers.

The invention comprises, in yet another form thereof, a surgical instrument for use in a computer assisted navigation system. The instrument includes a rotary member and a mounting assembly operably coupled to the rotary member wherein the rotary member and the mounting assembly are relatively rotatable about an axis. At least one reference element registerable in the computer assisted navigation system is disposed on the mounting assembly at a predetermined location. An anti-rotation feature disposed on the mounting assembly biases the mounting assembly toward an orientation wherein the at least one reference element is disposed vertically above the axis during relative rotation of the rotary member and the mounting assembly with the axis being horizontally disposed. The anti-rotation feature may be a counterweight secured to the mounting assembly diametrically opposite the reference element relative to the axis.

The invention comprises, in still another form thereof, a method of providing a rotary surgical tool for use in a computer assisted navigation system. The method includes providing a shaft and coupling a mounting assembly with the shaft wherein the mounting assembly and the shaft are relatively rotatable about an axis. The mounting assembly has disposed thereon at least one reference element that is registerable in the computer assisted navigation system. The method also includes rotating the shaft relative to the mounting assembly and simultaneously non-manually biasing the mounting assembly toward a desired orientation relative to the axis wherein the at least one reference element is disposed vertically above the axis when the axis is oriented horizontally. The biasing of the mounting assembly toward a desired orientation may include disposing a counterweight on the mounting assembly and gravitationally biasing the reference element. The method may also include the step of coaxially securing a rotatable tool to the shaft with a collet assembly.

An advantage of the present invention is that it provides a means for mounting a reference element registrable in a computer assisted navigation system on a surgical instrument having a rotary member and maintaining the reference element in a desired orientation relative to the surrounding environment during operation of the tool. This can allow the reference element to be positioned generally above the tool to facilitate maintaining a line of sight between the reference element and a sensor. The ability to maintain the reference element within the line of sight of a navigation sensor is of particular importance for some types of computer assisted navigation systems such as optical systems that detect light reflected from or generated by the reference elements.

Another advantage of the present invention is that it provides a collet assembly that allows the shank of a rotating tool to be firmly grasped and thereby limit any movement of the rotational axis of the tool relative to the at least one reference element which is used by the computer assisted navigational system to compute the position of the rotating tool.

### BRIEF DESCRIPTION OF THE DRAWINGS

The above mentioned and other features and objects of this invention, and the manner of attaining them, will become more apparent and the invention itself will be better understood by reference to the following description of an embodiment of the invention taken in conjunction with the accompanying drawings, wherein:
Figure 1 is an exploded view of a surgical instrument in accordance with the present invention.
Figure 2 is an exploded, partially cross sectional view of a rotary shaft and collet assembly.
Figure 3 is a cross sectional view of a mounting member.
Figure 4 is a partially cross sectional view of a quick-connect fitting.
Figure 5 is an end view of the mounting member.

Corresponding reference characters indicate corresponding parts throughout the several views. Although the exemplification set out herein illustrates an embodiment of the invention, the embodiment disclosed below is not intended to be exhaustive or to be construed as limiting the scope of the invention to the precise form disclosed.

### DESCRIPTION OF THE PRESENT INVENTION

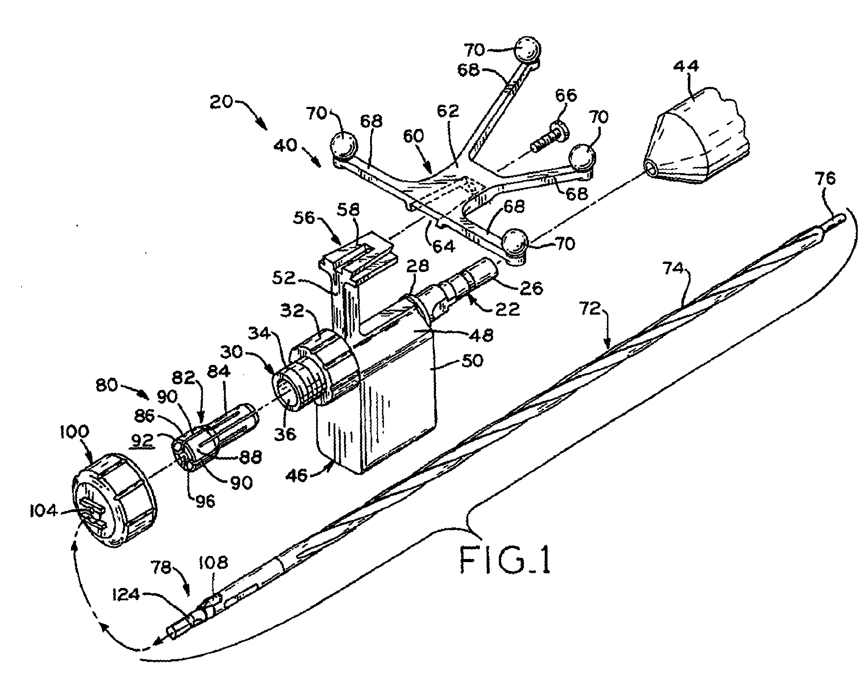

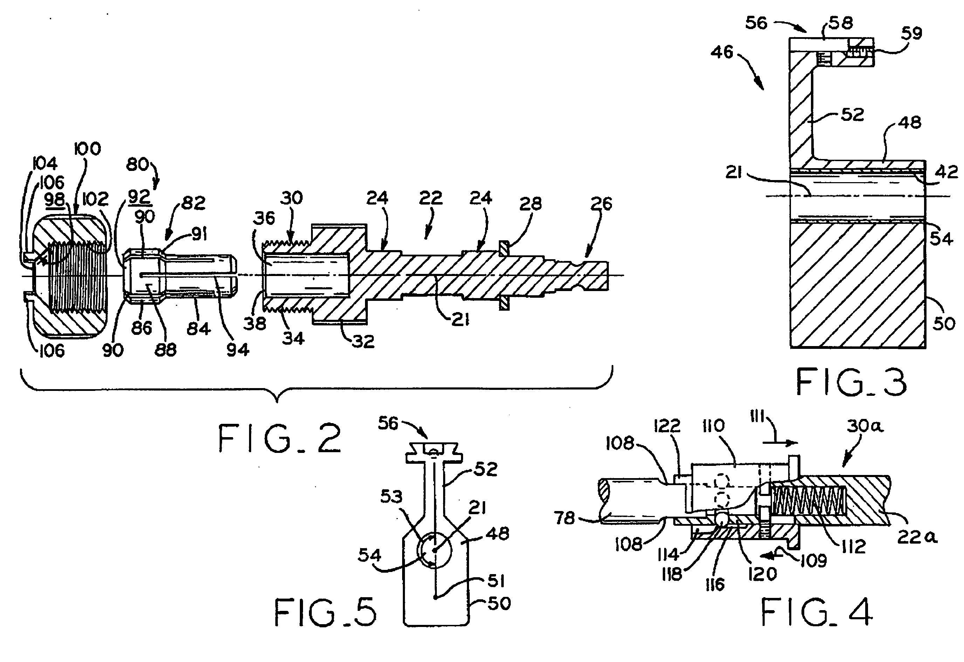

A surgical instrument 20 in accordance with the present invention is shown in Figure 1. Surgical instrument 20 includes a rotary member 22 which is rotationally engaged with mounting assembly 40. Rotary member 22 is best seen in Figures 1 and 2 and forms a shaft having two cylindrical portions 24 engaged with bearing sleeve insert 42 located in mounting assembly 40. Shaft 22 also includes a first end 26 which has a conventional shape for engagement with a surgical drill 44 or other powered or manual rotary driver. A washer-shaped retainer 28 is welded to shaft 22 to secure mounting assembly 40 on shaft 22 as discussed in greater detail below. Opposite first end 26 is second end 30 of shaft 22. Second end 30 includes an integrally formed radially enlarged grip portion 32 and a threaded shaft portion 34. An axially extending cylindrical opening 36 defines an opening 38 on the distal face of second end 30.

Mounting assembly 40 includes a mounting member 46 having a sleeve portion 48 and an integrally formed counterweight 50 and mounting stem 52. Sleeve portion 48 surrounds shaft 22 and defines a cylindrical opening 54 in which bearing sleeve 42 is located. The radially distal end 56 of mounting stem 56 has a male dovetail joint 58 and a threaded opening 59 for mounting reference array 60 thereon.

Reference array 60 includes an aluminum support structure 62 which forms a female dovetail joint 64 and four outwardly extending arms 68. Each of the support arms 68 has a reference element 70 are mounted thereon. In the illustrated embodiment, reference elements 70 are reflective spheres which are registerable in a computer assisted navigation system as discussed in greater detail below. A threaded fastener 66 securely is engaged with threaded opening 59 to firmly secure array 60 on mounting stem 52 after engaging dovetail joints 58, 64.

A collet assembly 80 is located at second end 30 and is used to secure a rotating surgical tool such as reamer 72 to shaft 22. Reamer 72 is a conventional reamer having a long shaft portion 74 with cutting threads and a blunt tip 76. Reamer 72 also includes a conventionally configured engagement shank 78. Collet assembly 80 includes a collet 82 having a small diameter portion 84 and a larger diameter portion 86. In the illustrated embodiment, collet 82 includes four flexible fingers 88 which are separated by gaps 90 and may be biased radially inwardly into the central void space defined by collet 82. Gaps 92 extend centrally down fingers 88 and enhance the flexibility of collet 82. A camming surface 92 is located at the distal ends of fingers 88 and is engageable with camming surface 98 of biasing member 100.

A second camming surface 91 on collet fingers 88 engages the surface defining opening 38 when smaller diameter portion 84 of collet is disposed into cylindrical bore 36. Larger diameter portion 86 extends outwardly from bore 36 and is circumscribed by biasing member 100. Biasing member 100 includes interior threads 102 which engage exterior threads 34 and as biasing member 100 is increasingly engaged with threads 34, camming surface 98 biases collet fingers 88 radially inwardly and toward opening 38. Engagement of opening 38 with camming surfaces 91 also biases collet fingers 88 inwardly toward the central void defined by collet 82.

Collet assembly 80 may thereby firmly engage shank 78 of reamer 72 when it is inserted through opening 104 of biasing member 100. Collet fingers 88 may also be used to firmly grip other rotatable tools. Biasing member 100 also includes 106 disposed on opposite sides of opening 104 which engage flats 108 located on shank 78. Shank 78 has a conventional configuration known as a Hudson connector/Trinkle adaptor. Collet fingers 88, however, may also be used with tools having alternative shaped shanks or engagement features.

An alternative second end 30a which may be used instead of collet assembly 80 is shown in Figure 4. This alternative connector has an outer sleeve 110 which surrounds shaft 22a. Shaft 22a is similar to shaft 22 except for second end 30a. A biasing member 112 biases sleeve 110 in the direction indicated by arrow 109. The interior surface of sleeve 110 has two portions which have different diameters. Disengagement portion 114 has defines a larger diameter than locking portion 116. Both portions 114 and 116 face locking balls 118 disposed in openings in hollow cylindrical portion 120 of shaft 22a. When sleeve 110 is disposed in the position illustrated in Figure 4, balls 118 are biased inwardly by inner surface 116 of sleeve 110 and into engagement with a circumferentially extending depression 124 on shank 78 to thereby lock shank 78 within shaft 22a. Balls 118 secure shank 78 to shaft 22a but do not prevent the relative rotation of shank 78 and shaft 22a. Projections 122 on shaft 22a engage flats 108 to prevent the relative rotation of shank 78 and shaft 22a. To dismount shank 78, sleeve 110 is moved in the direction indicated by arrow 111 and radially enlarged inner surface 114 allows locking balls 118 to disengage from shank 78.

The quick connect locking feature illustrated in Figure 4 includes four locking balls 118 to provide a relatively secure engagement between shaft 22a and the rotary tool engaged thereto. Manufacturing the quick connect fitting to relatively tight tolerances can also improve the engagement between the two shafts being joined. Collet assembly 80 located on shaft 22 also provides a relatively secure connection that maintains reamer 72 in a position in which its rotational axis is aligned with the axis 21 of shaft 22 and minimizes any movement of the rotational axis of reamer 72 relative to shaft 22 and mounting assembly 40, i.e., it inhibits wobbling of reamer 72.

By providing a relatively firmer connection between shaft 22, 22a and a rotating tool such as reamer 78, the tracking of the tool by a computer assisted navigational system may be improved by reducing the wobble of the tool relative to shaft 22, 22a. Oftentimes, conventional surgical drills have connections for engaging reamers or other rotating tools which allow some wobbling of the rotating tool. In such a situation, if a reference array were mounted to the housing of the drill the position of the rotating tool calculated by the navigation system will be inaccurate to the extent that the tool wobbles and departs from its assumed position relative to the reference array which is directly tracked by the navigation system.

As can be seen in Figure 1, shaft 22 and mounting assembly 40 are positioned between drill 44 and reamer 78 and any wobble created by the connection between drill 44 and first end 26 of shaft 22 does not affect the relative position of reference array 60 and reamer 78. Reamer 78, or other rotatable tool, is firmly fixed to shaft 22 to prevent or minimize relative movement of the tool.

Mounting assembly 40 is provided to position array 60 and reference elements 70 mounted thereon at a predefined relative position to the attached tool so that a computer navigation system tracking the positions of reference elements 70 can determine the position of the tool attached to second end 30. The relative axial movement of array 60 and second end 30, and any tool secured thereto, is prevented by positioning mounting assembly 40 between grip 32 and retainer 28. When assembling together shaft 22 and mounting assembly 40, mounting assembly 40 is positioned on shaft 22 and then retainer 28 is welded to shaft 22 to secure mounting assembly 40 between grip 32 and retainer 28 and prevent relative axial displacement of mounting assembly 40 and shaft 22.

For navigation systems which require there to be a clear line of sight between the reference elements being tracked and the sensors tracking the elements, such as an optical system wherein the sensors detect light either reflected or emitted by the reference elements, it is desirable that the reference elements be positioned above axis 21 to increase their visibility. The navigation system may not recognize array 60 if it were position below axis 21 in an "upside down" orientation. Thus, it is generally desirable to position array 60 vertically above axis 21.

Reference numeral 51 indicates the location of the center of gravity of the mounting assembly 40 and is shown in Figure 5. Center of gravity 51 is for the entire mounting assembly 40 which rotates relative to shaft 22 and thus includes array 60. As can also be seen in Figure 5, mounting stem 52 is disposed diametrically opposite (with respect to axis 21) counterweight portion 50. As described above reference array 60 is mounted on distal end 56 of mounting stem 52 which is located at a first angular position relative to axis 21. Center of gravity 51 defines a second angular position relative to axis 21 and, as shown by angle 53, the angular positions of array 60 and center of gravity 51 are separated by an angle of 180 degrees.

Because mounting assembly 40 is rotatable relative to shaft 22 and is not secured to any other part, gravitational forces acting on mounting assembly 40 will bias the center of gravity 51 of mounting assembly 40 toward a position directly below the rotational axis 21. The present invention utilizes a counterweight 50 which is radially spaced from axis 21 to control the position of center of gravity 51 of mounting assembly 40. Counterweight 50 is configured to position center of gravity 51 diametrically opposite array 60 and thereby gravitationally bias array 60 toward a position above axis 21. In the illustrated embodiment, mounting member 46, including counterweight portion 50, are relatively dense stainless steel and array 60 is relatively light aluminum. Other materials, however, may also be used to position center of gravity 51 in a desired location. Stated in terms of angular position relative to axis 21, to maintain a reference element 70 at a position at or above axis 21 when axis 21 is horizontally disposed, the angular positions of the reference element and the center of gravity relative to axis 21 must be separated by at least 90 degrees.

By using two raised cylindrical portions 24 to engage bearing sleeve 42 proximate its ends, mounting member 40 is rotatably mounted on shaft 22 in a stable manner and which limits the contact surface area between shaft 22 and bearing sleeve 42 to reduce frictional resistance to the relative rotation of shaft 22 and mounting assembly 40. In the illustrated embodiment, sleeve 42 is a teflon sleeve, however, other metallic and polymeric materials can be used to form sleeve 42. Alternative bearings having different designs could also be positioned between shaft 22 and mounting member 46, or, shaft could bear directly against mounting member 46.

Due to the presence of counterweight portion 50, array 60 will remain positioned above shaft 22 and axis 21 as shaft 22 is rotated by drill 44 or other rotary driver and in turn rotates reamer 78 or other rotary tool. Thus, the surgeon is not required to manually retain mounting assembly 40 in this desirable position. Counterweight 50 thereby acts as an anti-rotation feature on mounting assembly 40. An alternative embodiment of mounting assembly 40 could include an alternative anti-rotation feature such as an engagement arm adapted for engaging the housing of the drill or other non-rotating structure to prevent mounting assembly 40 from rotating with shaft 22. An advantage of counterweight 50 is that it provides an anti-rotation feature which is not dependent upon engagement with any other stationary structure. As used herein, an anti-rotation feature is a feature which inhibits the rotation of mounting assembly 40 about axis 21 relative to the surrounding environment but which still allows for the relative rotation of shaft 22 and mounting assembly 40.

As described above, array 60 is mounted on mounting arm 52 and includes four referencing elements 70. By providing at least three non-linearly positioned reference elements 70 on array 60, the determination of the position of these reference elements allows the computer assisted navigation system to calculate the position and orientation of reference array 60 and thereby also calculate the position and orientation of shaft 22 and a tool attached thereto.

As is known in the art, data concerning the fixed size and shape of a surgical instrument, such as reamer 78, which will be used in an image guided procedure can be determined pre-operatively to obtain a three dimensional model of the instrument or the relevant portions thereof. Additionally, the relevant dimensional data concerning an anatomical structure of interest, e.g., a femur, may be determined using data acquired from images of the anatomical structure to generate a data base representing a model of the anatomical structure. The model of the anatomical structure may be a three dimensional model which is developed by acquiring a series of two dimensional images of the anatomical structure. Alternatively, the model of the anatomical structure may be a set of two dimensional images having known spatial relationships or other data structure which can be used to convey information concerning the three dimensional form of the anatomical structure. The model of the anatomical structure may then be used to generate displays of the anatomical structure from various perspectives for preoperative planning purposes and intraoperative navigational purposes. A variety of technologies which may be employed to generate such a model of an anatomical structure are well known in the art and include computed tomography (CT), magnetic resonance imaging (MRI), positron emission tomography (PET), ultrasound scanning and fluoroscopic imaging technologies.

The model of the anatomical structure obtained by such imaging technologies can be used for the intraoperative guidance of a surgical instrument by facilitating the determination and display of the relative position and orientation of the surgical instrument with respect to the actual anatomical structure. For example, if the model of the anatomical structure is a set of two dimensional images having known spatial relationships, several such images may be simultaneously displayed during the surgical procedure. By also displaying the position of the surgical instrument in the images and displaying images taken from different perspectives, e.g., one image facilitating the display of instrument movement along the x and y coordinate axes and another image facilitating the display of instrument movement along the z axis, the individual images may together represent the movement of the surgical instrument in three dimensions relative to the anatomical structure.

For reference purposes, a coordinate system defined by the actual anatomical structure which is the subject of interest will be referred to herein as the anatomical coordinate system and a coordinate system defined by the model of the anatomical structure will be referred to as the image coordinate system.

Rigid anatomical structures, such as skeletal elements, are well suited for such image guided surgical techniques and individual skeletal elements may be used to define separate coordinate systems. The different rigid structures, e.g., skeletal elements, may be subject to relative movement, for example, the femur and acetabulum of a patient may be relatively moved during the surgical procedure and separate three dimensional models and coordinate systems may be created for the different skeletal elements. For example, during a hip replacement procedure, a three dimensional model of the femur defining a first coordinate system may be utilized during the preparation of the femur while a separate coordinate system defined by a three dimension model of the pelvis is utilized during the preparation of the acetabulum.

When using computer assisted navigation, also referred to as computer implemented image guidance, to conduct a surgical technique, the image coordinate system is registered with the anatomical coordinate system and the position of the surgical instrument or other tracked object is also registered within the image coordinate system. After the registration of both the actual anatomical structure and the surgical instrument, the relative position and orientation of the surgical instrument may be communicated to the surgeon by displaying together images of the anatomical structure and the instrument based upon the three dimensional models of the anatomical structure and instrument which were previously acquired.

Instruments registerable within a computer assisted navigation system and which could be employed or adapted for use as digitizing probes to engage a tool at a known location, such as tip 76 of reamer 72, and thereby calibrate the position of tip 76 relative to array 60 in the navigational system are described by Grimm et al. in a U.S. Patent Application entitled IMPLANT REGISTRATION DEVICE FOR SURGICAL NAVIGATION SYSTEM having attorney docket no. ZIM0166 filed on the same date as the present application, and by McGinley et al. in a U.S. Patent Application entitled SURGICAL NAVIGATION INSTRUMENT USEFUL IN MARKING ANATOMICAL STRUCTURES having attorney docket no. ZIM0167 filed on the same date as the present application.

Computer implemented image guidance systems which provide for the registration of an actual anatomical structure with a three dimensional model representing that structure together with the registration or localization of another object such as a surgical instrument or orthopedic implant within the image coordinate system to facilitate the display of the relative positions of the object and the actual anatomical structure are known in the art. Known methods of registering the anatomical structure with the image coordinate system include the use of implanted fiducial markers which are recognizable by one or more scanning technologies. Alternatively, implants which may be located by physically positioning a digitizing probe or similar device in contact or at a known orientation with respect to the implant. Instead of using fiducial implants, it may also be possible to register the two coordinate systems by aligning anatomical landmark features. U.S. Patent Nos. 6,236,875 B1 and 6,167,145 both describe methods of registering multiple rigid bodies and displaying the relative positions.

Tracking devices employing various technologies enabling the registration or localization of a surgical instrument and the tracking of the instrument motion with respect to the anatomical coordinate system, which has also been registered with the image coordinate system, are also known. For example, optical tracking systems which detect light from reflected or emitted by reflective targets or localizing emitters secured in a known orientation to the instrument are known for determining the position of the instrument and registering the position of the instrument within an image coordinate system representing a three dimensional model of an anatomical structure. For example, such a tracking system may take the form of a sensor unit having one or more lenses each focusing on separate charge coupled device (CCD) sensitive to infrared light. The sensor unit detects infrared light emitted by three or more non-linearly positioned light emitting diodes (LEDs) secured relative to the object. A processor analyzes the images captured by the sensor unit and calculates the position and orientation of the instrument. By registering the position of the sensing unit within the image coordinate system, the position of the instrument relative to the anatomical structure, which has also been registered with the image coordinate system, may be determined and tracked as the instrument is moved relative to the anatomical structure.

Alternative localizing systems may employ localizing emitters which emit an electromagnetic signal in the radio frequency or which emit visible light. Other types of localizing systems that could be used with the present invention employ referencing elements or other distinguishing elements which are radio-opaque. It is also possible to employ digitizing physical probes which are brought into physical contact with the object at predefined locations on the object to register the position of the object.

In the disclosed embodiment, the localizing system includes a light source and reference elements 70 reflect the light. The localizing system then detects the reflected light and computes the location of the individual reference elements 70 in a known manner. Reference elements 70 may be obtained from Northern Digital Inc. having a place of business at 103 Randall Dr., Waterloo, Onterio, Canada, N2V1C5. Northern Digital Inc. supplies image guidance systems under the brand names Optotrak® and Polaris® which may be used with the present invention. The present invention may also be used with other computer assisted navigation systems such as those described above or otherwise known in the art For example, Medtronic, Inc. headquartered in Minneapolis, Minnesota manufactures and sells various computer assisted surgical navigation systems under the trademark StealthStation® such as the FluoroNav^{™} Virtual Fluoroscopy System which could also be adapted for use with the present invention.

An alternative embodiment of the present invention could be employed with a computer assisted navigation system which utilizes magnetic fields instead of optical tracking to determine the position and orientation of the tracked object. A variety of referencing elements which are used with magnetic fields which could be adapted for use with the present invention are known in the art. For example, known systems using magnetic fields to determine the position and orientation of an object are described by U.S. Pat. Nos. 5,913,820; 6,381,485 B1; 6,402,762 B2; 6,474,341 B1; 6,493,573 B1; and 6,499,488 B1.

By generating a magnetic field of known properties in the operative area and sensing the field with mutually perpendicular wire loops, the position and orientation of the reference elements defined by the wire loops and the object, such as a surgical instrument, attached thereto may be calculated. The detemrination of the position and orientation of such mutually perpendicularly oriented field sensors is known in the art. It is also known to use a single wire loop to form a single field sensor and determine its position and orientation by generating magnetic fields from a plurality of locations.

While this invention has been described as having an exemplary design, the present invention may be further modified. This application therefore intended to cover any variations, uses, or adaptations of the invention using its general principles.

## Claims

1. A surgical instrument (20) for use in a computer assisted navigation system, **characterised in that** said instrument comprises:
at least one reference element (70) registerable in the computer assisted navigation system,:
a mounting assembly (40) defining an axis (Z1) and rotatably mounted on the instrument(22), said at least one reference element (70) positionable on said mounting assembly (40) in a predetermined location, said predetermined location defining a first angular position relative to said axis (2t); and
a counterweight (50) disposed on said mounting assembly (40) and radially outwardly spaced from said axis (21), said counterweight(50) defining a second angular position relative to said axis (21), said first and second angular positions separated by at least 90 degrees.

2. The surgical instrument (20) of Claim 1 **characterised in that** said mounting assembly (40) further comprises a radially outwardly extending mounting stem (52), said stem (52) disposed substantially diametrically opposite said counterweight (50) relative to said axis (21), said at least one reference element (70) being positioned on a radially distal end of said mounting stem (52).

3. The surgical instrument(20) of Claim 1 or Claim 2 **characterised in that** said mounting assembly (40) includes a sleeve portion (48) defining a cylindrical opening (54) and said counterweight (50) is integrally formed with said sleeve portion (48).

4. The surgical instrument(20) of any preceding claim **characterised in that** said at least one reference element (70) comprises at least three non-linearly positioned reference elements (70).

5. The surgical instrument(20) of any preceding claim **characterised in that** it further comprises a rotary member (22) having a cylindrical shaft portion (24) rotationally engaged with said mounting assembly (40).

6. The surgical instrument(20) of Claim 5 **characterised in that** said rotary member (22) further comprises a collet assembly (80) disposed at an end (30) thereof, said collet assembly (80) including a collet (82) and a biasing member (100), said collet (82) defining a central void and having a plurality of fingers (88) biasable inwardly relative to said void, said biasing member (100) biasingly engageable with said plurality of collet fingers (88) and wherein said biasing member (100) is securable in a position biasing said plurality of fingers (88) inwardly relative to said central void.

7. The surgical instrument (20) of Claim 6 **characterised in that** said end (30) is defined by a cylindrical shaft (34) having exterior threads and an axially disposed opening (38), said collet (82) partially positioned in said opening (38), at least a portion of said plurality of collet fingers projecting from said opening (38), said biasing member (100) threadingly engaged with said exterior threads and circumscribing said projecting portion of said plurality of collet fingers (88).

8. A surgical instrument (20) as claimed in any preceding claim **characterised in that** said counterweight (50) defining a center of gravity spaced radially outwardly from said axis (21) wherein said reference element (70) defines a first angular position relative to said axis (21) and said center of gravity (51) defines a second angular position relative to said axis (21), said first and second angular positions separated by at least 90 degrees.

9. The surgical instrument (20) of Claim 8 **characterised in that** said mounting assembly (40) comprises a sleeve portion (48) defining a cylindrical opening (36), said mounting assembly (40) having a counterweight portion (50) disposed radially outwardly from said axis (21), said at least one reference element (70) disposed substantially diametrically opposite said counterweight portion (50) relative to said axis (21).

10. The surgical instrument (20) of Claim 9 **characterised in that** said mounting assembly (40) further includes a mounting stem (52) extending radially outwardly from said sleeve portion (48) and disposed substantially diametrically opposite said counterweight portion (50) relative to said axis (21), said reference element (70) removably mountable on a radially distal end (50) of said mounting stem (52).

11. The surgical instrument (20) of Claim 8 **characterised in that** said at least one reference element (70) comprises at least three non-linearly positioned reference elements (70).

12. The surgical instrument (20) of Claim 8 **characterised in that** the instrument (20) comprises a rotary member (22) including a cylindrical shaft portion (24) rotatably engaged with said mounting assembly (40).

13. The surgical instrument(20) of Claim 8 **characterised in that** it further comprises a rotational driver (14) detachably securable to said first end (26).

14. The surgical instrument (20) of Claim 8 **characterised in that** it further comprises a rotatable tool (72) detachably securable to said second end (30).

15. The surgical instrument (20) of any preceding claim **characterised in that** it further comprises a surgical tool (72) having a shank (74), said shank (74) being insertable into said central void, said shank (74) being rotationally fixedly engageable by said inwardly biasable plurality of collet fingers. (88).

16. A surgical instrument (20) as claimed in Claim 5 **characterised in that** said instrument comprising:
an anti-rotation feature (50) disposed on said mounting assembly (40) biasing said mounting assembly (40) toward an orientation wherein said reference element (70) is disposed vertically above said axis (21) during relative rotation of said rotary member (22) and said mounting assembly (40) about said axis (21) horizontally disposed.

17. The surgical instrument (20) of Claim 16 **characterised in that** said anti-rotation feature(50) comprises a counterweight (50) secured to said mounting assembly (40) diametrically opposite said at least one reference element (70) relative to said axis (21).

18. The surgical instrument of any preceding claim **characterised in that** it further comprises a bearing sleeve insert (42) positioned between said cylindrical shaft portion (24) of said rotary member (22) and said sleeve portion (48) of said mounting assembly.

19. The surgical instrument of Claim 5 **characterised in that** said rotary member (22) is defined by a cylindrical shaft (34) having exterior threads and an axially disposed opening (36) wherein a collet (82) is partially positioned in said opening (36), with at least a portion of a plurality of collet fingers (88) projecting from said opening (36), and said biasing member (100) is threadingly engaged with said exterior threads and circumscribes said projecting portion of said plurality of collet fingers (88).

20. The surgical instrument of Claim 1 chracterised in that it further comprises a rotary member (22) having a cylindrical shaft portion (24) engaged for relative rotation with respect to a sleeve portion (48) of said mounting assembly (40); and
a locking assembly for temporarily locking said mounting assembly (40) with respect to said rotary member (22) to prevent relative rotation therebetween.

21. The surgical instrument of Claim 20 **characterised in that** said locking assembly includes a shaft retainer (28) positioned on said rotary member (22);
a locking lever pivotably attached to said mounting assembly (40) whereby said locking lever is configured to be pivoted to engage a portion of said shaft retainer (28), thereby preventing relative rotation between said rotary member (22) and said mounting assembly (40).

22. A method of providing a rotary surgical tool for use in a computer assisted navigation system **characterised in that** said method comprises:
providing a shaft (22);
coupling a mounting assembly (40) with said shaft (72) wherein said mounting assembly (40) and said shaft (22) are relatively rotatable about an axis (21), said mounting assembly (40) having at least one reference element (70) registerable in a computer assisted navigation system disposed thereon; and
rotating said shaft (22) relative to said mounting assembly (40) and simultaneously non-manually biasing said mounting assembly (40) toward a desired orientation relative to said axis (21) wherein said at least one reference element (70) is disposed vertically above said axis (21) when said axis (21) is oriented horizontally.

23. The method of Claim 22 **characterised in that** said biasing of said mounting assembly (40) toward a desired orientation comprises disposing a counterweight (50) on said mounting assembly (40) and gravitationally biasing said at least one reference element (70).

24. The method of claim 23 **characterised in that** said at least one reference element (70) comprises at least three non-linearly disposed reference elements (70).

25. The method of Claim 23 **characterised in that** it further comprises the step of coaxially securing a rotatable tool (72) to said shaft (22) with a collet assembly (80).

## Patentansprüche

1. Chirurgisches Instrument (20) zur Verwendung in einem computergestützten Navigationssystem, **dadurch gekennzeichnet, daß** das Instrument aufweist:
mindestens ein Bezugselement (70), das in dem computergestützten Navigationssystem registrierbar ist;
eine Befestigungsanordnung (40), die eine Achse (21) definiert und drehbar auf dem Instrument (22) angeordnet ist, wobei das mindestens eine Bezugselement (70) auf der Befestigungsanordnung (40) an einer vorbestimmten Stelle positionierbar ist, wobei die vorbestimmte Stelle eine erste Winkelposition relativ zu der Achse (21) definiert; und
ein Gegengewicht (50), das auf der Befestigungsanordnung (40) angeordnet ist und radial nach außen von der Achse (21) beabstandet ist, wobei das Gegengewicht (50) eine zweite Winkelposition relativ zu der Achse (21) definiert, wobei die erste und zweite Winkelposition um mindestens 90° getrennt sind.

2. Chirurgisches Instrument (20) nach Anspruch 1, **dadurch gekennzeichnet, daß** die Befestigungsanordnung (40) ferner einen sich radial nach außen erstreckenden Befestigungsfuß (52) aufweist, wobei der Fuß (52) im wesentlichen diametral gegenüber dem Gegengewicht (50) relativ zu der Achse (21) angeordnet ist, wobei das mindestens eine Bezugselement (70) an einem radial distalen Ende des Befestigungsfußes (52) positioniert ist.

3. Chirurgisches Instrument (20) nach Anspruch 1 oder 2, **dadurch gekennzeichnet, daß** die Befestigungsanordnung (40) einen Hülsenabschnitt (48) aufweist, der eine zylindrische Öffnung (54) definiert, und das Gegengewicht (50) einstückig mit dem Hülsenabschnitt (48) ausgebildet ist.

4. Chirurgisches Instrument (20) nach einem der vorhergehenden Ansprüche, **dadurch gekennzeichnet, daß** das mindestens eine Bezugselement (70) mindestens drei nichtlinear positionierte Bezugselemente (70) umfaßt.

5. Chirurgisches Instrument (20) nach einem der vorhergehenden Ansprüche, **dadurch gekennzeichnet, daß** es ferner ein drehbares Teil (22) mit einem zylindrischen Wellenabschnitt (24) aufweist, der mit der Befestigungsanordnung (40) in Dreheingriff ist.

6. Chirurgisches Instrument (20) nach Anspruch 5, **dadurch gekennzeichnet, daß** das drehbare Teil (22) ferner eine Spannhülsenanordnung (80) aufweist, die an einem Ende (30) desselben angeordnet ist, wobei die Spannhülsenanordnung (80) eine Spannhülse (82) und ein Spannteil (100) aufweist, wobei die Spannhülse (82) einen mittigen Hohlraum definiert und eine Vielzahl von Fingern (88) hat, die nach innen relativ zu dem Hohlraum spannbar sind, wobei das Spannteil (100) mit der Vielzahl von Spannhülsenfingern (88) spanneingriffsfähig ist und wobei das Spannteil (100) in einer Position befestigbar ist, die die Vielzahl von Fingern (88) nach innen relativ zu dem mittigen Hohlraum spannt.

7. Chirurgisches Instrument (20) nach Anspruch 6, **dadurch gekennzeichnet, daß** das Ende (30) durch eine zylindrische Welle (34) mit Außengewinde und eine axial angeordnete Öffnung (38) definiert ist, wobei die Spannhülse (82) teilweise in der Öffnung (38) positioniert ist, wobei zumindest ein Abschnitt der Vielzahl von Spannhülsenfingern aus der Öffnung (38) vorsteht, wobei das Spannteil (100) mit dem Außengewinde in Gewindeeingriff ist und den vorstehenden Abschnitt der Vielzahl von Spannhülsenfingern (88) eingrenzt.

8. Chirurgisches, Instrument (20) nach einem der vorhergehenden Ansprüche, **dadurch gekennzeichnet, daß** das Gegengewicht (50) einen Schwerpunkt definiert, der radial nach außen von der Achse (21) beabstandet ist, wobei das Bezugselement (70) eine erste Winkelposition relativ zu der Achse (21) definiert und der Schwerpunkt (51) eine zweite Winkelposition relativ zu der Achse (21) definiert, wobei die erste und zweite Winkelposition um mindestens 90° getrennt sind.

9. Chirurgisches Instrument (20) nach Anspruch 8, **dadurch gekennzeichnet, daß** die Befestigungsanordnung (40) einen Hülsenabschnitt (48) aufweist, der eine zylindrische Öffnung (36) definiert, wobei die Befestigungsanordnung (40) einen Gegengewichtabschnitt (50) hat, der von der Achse (21) radial nach außen angeordnet ist, wobei das mindestens eine Bezugselement (70) im wesentlichen diametral gegenüber dem Gegengewichtabschnitt (50) relativ zu der Achse (21) angeordnet ist.

10. Chirurgisches Instrument (20) nach Anspruch 9, **dadurch gekennzeichnet, daß** die Befestigungsanordnung (40) ferner einen Befestigungsfuß (52) aufweist, der sich von dem Hülsenabschnitt (48) radial nach außen erstreckt und im wesentlichen diametral gegenüber dem Gegengewichtabschnitt (50) relativ zu der Achse (21) angeordnet ist, wobei das Bezugselement (70) lösbar an einem radial distalen Ende (50) des Befestigungsfußes (52) befestigbar ist.

11. Chirurgisches Instrument (20) nach Anspruch 8, **dadurch gekennzeichnet, daß** das mindestens eine Bezugselement (70) mindestens drei nichtlinear positionierte Bezugselemente (70) umfaßt.

12. Chirurgisches Instrument (20) nach Anspruch 8, **dadurch gekennzeichnet, daß** das Instrument (20) ein drehbares Teil (22) mit einem zylindrischen Wellenabschnitt (24) aufweist, der mit der Befestigungsanordnung (40) in Dreheingriff ist.

13. Chirurgisches Instrument (20) nach Anspruch 8, **dadurch gekennzeichnet, daß** es ferner einen drehbaren Mitnehmer (14) aufweist, der lösbar an dem ersten Ende (26) befestigbar ist.

14. Chirurgisches Instrument (20) nach Anspruch 8, **dadurch gekennzeichnet, daß** es ferner ein drehbares Werkzeug (72) aufweist, das lösbar an dem zweiten Ende (30) befestigbar ist.

15. Chirurgisches Instrument (20) nach einem der vorhergehenden Ansprüche, **dadurch gekennzeichnet, daß** es ferner ein chirurgisches Werkzeug (72) mit einem Schaft (74) aufweist, wobei der Schaft (74) in den mittigen Hohlraum einfügbar ist, wobei der Schaft (74) durch die nach innen spannbare Vielzahl von Spannhülsenfingern (88) verdrehsicher eingriffsfähig ist.

16. Chirurgisches Instrument (20) nach Anspruch 5, **dadurch gekennzeichnet, daß** das Instrument aufweist:
ein Verdrehsicherungsmerkmal (50), das an der Befestigungsanordnung (40) eingerichtet ist und die Befestigungsanordnung (40) in einer Ausrichtung spannt, bei der das Bezugselement (70) während einer relativen Drehung des drehbaren Teils (22) vertikal über der Achse (21) angeordnet ist und die Befestigungsanordnung (40) um die Achse (21) horizontal angeordnet ist.

17. Chirurgisches Instrument (20) nach Anspruch 16, **dadurch gekennzeichnet, daß** das Verdrehungssicherungsmerkmal (50) ein Gegengewicht (50) aufweist, das an der Befestigungsanordnung (40) diametral gegenüber dem mindestens einen Bezugselement (70) relativ zu der Achse (21) befestigt ist.

18. Chirurgisches Instrument nach einem der vorhergehenden Ansprüche, **dadurch gekennzeichnet, daß** es ferner einen Lagerhülseneinsatz (42) aufweist, der zwischen dem zylindrischen Wellenabschnitt (24) des drehbaren Teils (22) und dem Hülsenabschnitt (48) der Befestigungsanordnung positioniert ist.

19. Chirurgisches Instrument nach Anspruch 5, **dadurch gekennzeichnet, daß** das drehbare Teil (22) durch eine zylindrische Welle (34) mit Außengewinde und eine axial angeordnete Öffnung (36) definiert ist, wobei eine Spannhülse (82) teilweise in der Öffnung (36) positioniert ist, wobei mindestens ein Abschnitt einer Vielzahl von Spannhülsenfingern (88) aus der Öffnung (36) vorsteht und das Spannteil (100) mit dem Außengewinde in Gewindeeingriff ist und den vorstehenden Abschnitt der Vielzahl von Spannhülsenfingern (88) eingrenzt.

20. Chirurgisches Instrument nach Anspruch 1, **dadurch gekennzeichnet, daß** es ferner ein drehbares Teil (22) mit einem zylindrischen Wellenabschnitt (24) aufweist, der zur relativer Drehung in bezug auf einen Hülsenabschnitt (48) der Befestigungsanordnung (40) eingerückt ist; und
eine Verriegelungsanordnung zum vorübergehenden Verriegeln der Befestigungsanordnung (40) in bezug auf das drehbare Teil (22), um eine relative Drehung zwischen diesen zu verhindern.

21. Chirurgisches Instrument nach Anspruch 20, **dadurch gekennzeichnet, daß** die Verriegelungsanordnung eine Wellensicherungsring (28) aufweist, der auf dem drehbaren Teil (22) positioniert ist;
einen Verriegelungshebel, der drehbar an der Befestigungsanordnung (40) angebracht ist, wobei der Verriegelungshebel so konfiguriert ist, daß er schwenkbar ist, um mit einem Abschnitt dem Wellensicherungsring (28) in Eingriff zu treten, wobei eine relative Bewegung zwischen dem drehbaren Teil (22) und der Befestigungsanordnung (40) verhindert wird.

22. Verfahren zur Bereitstellung eines drehbaren chirurgischen Werkzeugs zur Verwendung in einem computergestützten Navigationssystem, **dadurch gekennzeichnet, daß** das Verfahren aufweist:
Bereitstellen einer Welle (22);
Koppeln einer Befestigungsanordnung (40) mit der Welle (22), wobei die Befestigungsanordnung (40) und die Welle (22) um eine Achse (21) relativ drehbar sind, wobei die Befestigungsanordnung (40) mindestens ein Bezugselement (70) hat, das in einem daran angeordneten computergestützten Navigationssystem registrierbar ist; und
Drehen der Welle (22) relativ zu der Befestigungsanordnung (40) und gleichzeitiges nichtmanuelles Spannen der Befestigungsanordnung (40) in einer gewünschten Ausrichtung relativ zu der Achse (21), bei der das mindestens eine Bezugselement (70) vertikal über der Achse (21) angeordnet ist, wenn die Achse (21) horizontal ausgerichtet ist.

23. Verfahren nach Anspruch 22, **dadurch gekennzeichnet, daß** das Spannen der Befestigungsanordnung (40) in einer gewünschten Ausrichtung aufweist: Anordnen eines Gegengewichts (50) an der Befestigungsanordnung (40) und schwerkraftbedingtes Spannen des mindestens einen Bezugselements (70).

24. Verfahren nach Anspruch 23, **dadurch gekennzeichnet, daß** mindestens ein Bezugselement (70) mindestens drei nichtlinear angeordnete Bezugselemente (70) umfaßt.

25. Verfahren nach Anspruch 23, **dadurch gekennzeichnet, daß** es ferner den Schritt aufweist: koaxiales Befestigen eines drehbaren Werkzeugs (72) an der Welle (22) mittels einer Spannhülsenanordnung (80).

## Revendications

1. Instrument chirurgical (20) pour utilisation dans un système de navigation assisté par ordinateur, **caractérisé en ce que** ledit instrument comprend :
au moins un élément de référence (70) enregistrable dans le système de navigation assisté par ordinateur ;
un ensemble de montage (40) définissant un axe (21) monté avec faculté de rotation sur l'instrument (22), ledit au moins un élément de référence (70) étant positionnable sur ledit ensemble de montage (40) à un emplacement prédéterminé, ledit emplacement prédéterminé définissant une première position angulaire par rapport audit axe (21) ; et
un contrepoids (50) disposé sur ledit ensemble de montage (40) et espacé radialement vers l'extérieur dudit axe (21), ledit contrepoids (50) définissant une seconde position angulaire par rapport audit axe (21), lesdites première et seconde positions angulaires étant séparées par au moins 90 degrés.

2. Instrument chirurgical (20) selon la revendication 1, **caractérisé en ce que** ledit ensemble de montage (40) comprend, en outre, une tige de montage s'étendant radialement vers l'extérieur (52), ladite tige (52) étant disposée sensiblement opposée dans le sens du diamètre audit contrepoids (50) par rapport audit axe (21), ledit au moins un élément de référence (70) étant positionné sur une extrémité radialement distale de ladite tige de montage (52) .

3. Instrument chirurgical (20) selon la revendication 1 ou 2, **caractérisé en ce que** ledit ensemble de montage (40) inclut une partie de manchon (48) définissant une ouverture cylindrique (54) et ledit contrepoids (50) est formé solidairement avec ladite partie de manchon (48).

4. Instrument chirurgical (20) selon l'une quelconque des revendications précédentes, **caractérisé en ce que** ledit au moins un élément de référence (70) comprend au moins trois éléments de référence (70) positionnés de manière non linéaire.

5. Instrument chirurgical (20) selon l'une quelconque des revendications précédentes, **caractérisé en ce qu'**il comprend, en outre, un élément rotatif (22) ayant une partie d'arbre cylindrique (20 engagée en rotation avec ledit ensemble de montage (40).

6. Instrument chirurgical (20) selon la revendication 5, **caractérisé en ce que** ledit élément rotatif (22) comprend, en outre, un ensemble de collier (80) disposée à une de ses extrémités (30), ledit ensemble de collier (80) incluant un collier (82) et un élément de sollicitation (100), ledit collier (82) définissant un vide centrale et ayant une pluralité de doigts (88) qui peuvent être sollicités vers l'intérieur par rapport audit vide, ledit élément de sollicitation (100) pouvant s'engager par sollicitation avec ladite pluralité des doigts de collier (88) et dans lequel ledit élément de sollicitation (100) est fixée à une position sollicitant ladite pluralité des doigts (88) vers l'intérieur par rapport audit vide central.

7. Instrument chirurgical (20) selon la revendication 6, **caractérisé en ce que** ladite extrémité (30) est définie par un arbre cylindrique (31) ayant des filets extérieurs et une ouverture disposée axialement (38), ledit collier (82) étant positionné partiellement dans ladite ouverture (38), au moins une partie de ladite pluralité des doigts de collier dépassant de ladite ouverture (38), ledit élément de sollicitation (100) étant engagé par filets avec les filets extérieurs circonvenant ladite partie dépassant de ladite pluralité des doigts de collier (88).

8. Instrument chirurgical (20) selon l'une quelconque des revendications précédentes, **caractérisé en ce que** ledit contrepoids (50) définit un centre de gravité espacé radialement vers l'extérieur dudit axe (21), dans lequel ledit élément de référence (70) définit une première position angulaire par rapport audit axe (21) et ledit centre de gravité (51) définit une seconde position angulaire par rapport audit axe (21), lesdites première et seconde positions angulaires étant séparées par au moins 90 degrés.

9. Instrument chirurgical (20) selon l'une quelconque des revendications précédentes, **caractérisé en ce que** ledit ensemble de montage (40) comprend une partie de manchon (48) définissant une ouverture cylindrique (36), ledit ensemble de montage (40) ayant une partie de contrepoids (50) disposée radialement vers l'extérieur par rapport audit axe (21), ledit au moins un élément de référence (70) étant disposé sensiblement opposé dans le sens du diamètre à ladite partie de contrepoids (50), par rapport audit axe (21).

10. Instrument chirurgical (20) selon la revendication 9, **caractérisé en ce que** ledit ensemble de montage (40) inclut, en outre, une tige de montage (52) s'étendant radialement vers l'extérieur depuis ladite partie de manchon (48) et disposée de manière sensiblement opposée dans le sens du diamètre à ladite partie de contrepoids (50) par rapport audit axe (21), ledit élément de référence (70) pouvant être monté de manière amovible sur une extrémité radialement distale (50) de ladite tige de montage (52).

11. Instrument chirurgical (20) selon la revendication 8, **caractérisé en ce que** ledit au moins un élément de référence (70) comprend au moins trois éléments de référence (70) positionnés de manière non linéaire.

12. Instrument chirurgical (20) selon la revendication 8, **caractérisé en ce que** l'instrument (20) comprend un élément rotatif (22) incluant une partie d'arbre cylindrique (24) engagée en rotation avec ledit ensemble de montage (40).

13. Instrument chirurgical (20) selon la revendication 8, **caractérisé en ce qu'**il comprend, de plus, un dispositif d'entraînement en rotation (14) pouvant être fixé de manière détachable à ladite première extrémité (26).

14. Instrument chirurgical (20) selon la revendication 8, **caractérisé en ce qu'**il comprend, en outre, un outil rotatif (72) pouvant être fixé de manière détachable à ladite seconde extrémité (30).

15. Instrument chirurgical (20) selon l'une quelconque des revendications précédentes, **caractérisé en ce qu'**il comprend, en outre, un outil chirurgical (72) ayant une queue (74), ladite queue (70) pouvant être insérée dans ledit vide central, ladite queue (74) pouvant être engageable de manière fixe en rotation par ladite pluralité de doigts de collier (88) pouvant être sollicités vers l'intérieur.

16. Instrument chirurgical (20) selon la revendication 5, **caractérisé en ce que** ledit instrument comprend :
un composant anti-rotation (50) disposée sur ledit ensemble de montage (40) sollicitant ledit ensemble de montage (40) vers une orientation dans laquelle ledit élément de référence (70) est disposé verticalement au-dessus dudit axe (21) pendant la rotation relative dudit élément rotatif (22) et dudit ensemble de montage (40) autour dudit axe (21) disposée horizontalement.

17. Instrument chirurgical (20) selon la revendication 16, **caractérisé en ce que** ledit composant anti-rotation (50) comprend un contrepoids (50) fixé audit ensemble de montage (40) étant opposé dans le sens du diamètre audit au moins un élément de référence (70) par rapport audit axe (21).

18. Instrument chirurgical selon l'une quelconque des revendications précédentes, **caractérisé en ce qu'**il comprend, en outre, un insert de manchon de palier (42) positionné entre ladite partie d'arbre cylindrique (24) dudit élément rotatif (22) et ladite partie de manchon (48) dudit ensemble de montage.

19. Instrument chirurgical selon la revendication 5, **caractérisé en ce que** ledit élément rotatif (22) est défini par un arbre cylindrique (34) ayant des filets extérieurs et une ouverture disposée axialement (36), dans laquelle un collier (82) est positionné partiellement dans ladite ouverture (36), avec au moins une partie de la pluralité des doigts de collier (88) dépassant de ladite ouverture (36), et ledit élément de sollicitation (100) est engagé par filets avec lesdits filets extérieurs et circonscrit ladite partie dépassante de ladite pluralité des doigts de collier (88).

20. Instrument chirurgical selon la revendication 1, **caractérisé en ce qu'**il comprend, de plus, un élément rotatif (22) ayant une partie d'arbre cylindrique (24) engagée pour rotation relative par rapport à une partie de manchon (48) dudit ensemble de montage (40) ; et
un ensemble de verrouillage pour verrouiller temporairement ledit ensemble de montage (40) par rapport audit élément rotatif (22) pour empêcher une rotation relative entre eux.

21. Instrument chirurgical selon la revendication 20, **caractérisé en ce que** ledit ensemble de verrouillage inclut un dispositif de retenue d'arbre (28) positionné sur ledit élément rotatif (22) ;
un levier de verrouillage fixé en pivot audit ensemble de montage (40) moyennant quoi ledit levier de verrouillage est configuré pour être mis à pivoter pour engager une partie dudit dispositif de retenue d'arbre (28), empêchant de ce fait la rotation relative entre ledit élément rotatif (22) et ledit ensemble de montage (40).

22. Procédé de fourniture d'un outil chirurgical rotatif pour utilisation dans un système de navigation assisté par ordinateur **caractérisé en ce que** le procédé comprend :
fournir un arbre (22) ;
coupler un ensemble de montage (40) audit arbre (22), dans lequel ledit ensemble de montage (40) et ledit arbre (22) peuvent tourner relativement autour d'un axe (21), ledit ensemble de montage (40) ayant au moins un élément de référence (70) pouvant être enregistré dans un système de navigation assisté par ordinateur disposé sur celui-ci ; et
faire tourner ledit arbre (22) par rapport audit ensemble de montage (40) et solliciter simultanément de manière non manuelle ledit ensemble de montage (40) vers une orientation désirée par rapport audit axe (21), dans lequel ledit au moins un élément de référence (70) est disposé verticalement au-dessus dudit axe (21) lorsque ledit axe (21) est orienté horizontalement.

23. Procédé selon la revendication 22, **caractérisé en ce que** ladite sollicitation dudit ensemble de montage (40) vers une orientation désirée comprend de disposer un contrepoids (50) sur ledit ensemble de montage (40) et de solliciter par pesanteur ledit au moins un élément de référence (70).

24. Procédé selon la revendication 23, **caractérisé en ce que** ledit au moins un élément de référence (70) comprend au moins trois éléments de référence (70) disposés de manière non linéaire.

25. Procédé selon la revendication 23, **caractérisé en ce qu'**il comprend, en outre, l'étape consistant à fixer de manière coaxiale un outil rotatif (72) audit arbre (22) avec un ensemble de collier (80).
